# EUROPEAN PATENT APPLICATION

(11) **EP 2 497 769 A1**
(43) Date of publication of application: **12.09.2012**
(21) Application number: 10828355.7
(22) Date of filing: 05.11.2010
(51) Int. Cl.: C07D 471/04

(54) **PYRROLOQUINOLINE QUINONE IN FREE FORM**

(30) Priority: 06.11.2009 JP 2009255056
(71) Applicant: Mitsubishi Gas Chemical Company, Inc., Tokyo 100-8324 (JP)
(72) Inventor: IKEMOTO Kazuto, Niigata-shi Niigata 950-3112 (JP); EDAHIRO Junichi, Niigata-shi Niigata 950-3112 (JP)
(74) Representative: Müller-Boré & Partner Patentanwälte
(86) International application number: PCT/JP2010/069734
(87) International publication number: WO 2011/055796

(57) **Abstract**

An object of the present invention is to provide a method for conveniently producing pyrroloquinoline quinone in the free form without any organic solvent or ion-exchange resin and highly-pure crystals thereof. According to the present invention, there is provided a production method of pyrroloquinoline quinone in the free form and highly-pure crystals thereof, wherein the production method comprises preparing a solution having a pH of 1.5 or less by dissolving an alkali metal salt of pyrroloquinoline quinone to obtain a precipitate.

## Description

### CROSS-REFERENCE TO RELATED APPLICATION

This application enjoys the benefit of Japanese Patent Application No. 2009-255056, filed on November 6, 2009. The disclosure of the above application is incorporated herein by reference.

### TECHNICAL FIELD

This invention relates to a production method of pyrroloquinoline quinone in the free form and highly-pure crystals obtained therefrom.

### BACKGROUND ART

Pyrroloquinoline quinone (hereinafter sometimes referred to as "PQQ") has been proposed as a possible new vitamin (for example, Nature, vol. 422, 24 April, 2003, p832),and has attracted much attention as a useful material for dietary supplements, cosmetics, etc. PQQ is present not only in bacteria but also in eukaryotic molds and yeasts and plays an important role as a coenzyme. Also, PQQ has been found to have many physiological activities such as cell growth-promoting activity, anti-cataract activity, hepatic disease-preventing and therapeutic activity, wound healing activity, antiallergic activity, reverse transcriptase-inhibiting activity, glyoxalase I-inhibiting activity - anticancer activity, and the like. This PQQ can be obtained by subjecting PQQ obtained by methodologies such as organic chemical syntheses (JACS, vol. 103, pp 5599-5600 (1981)) and fermentation processes (Japanese Patent Application Laid-Open Publication No. 01-218597) to chromatography and concentrating the PQQ fraction in the effluent to crystallize PQQ by crystallization (Japanese Patent No. 2072284), followed by drying the crystallized PQQ. Depending upon the pH and crystallization conditions used, the PQQ crystals thus obtained are generally in the form of an alkali metal salt.

PQQ is required to be in the free form in order to use it as a raw material for synthesizing its derivatives or as a product dissolved in an organic solvent which does not dissolve its alkali metal salt. In JACS, vol. 103, pp 5599-5600 (1981), the PQQ crystals are obtained by adjusting the pH to 2.5. As a result of extensive studies of the present inventors, however, it has been found that in such pH range PQQ is present not in the free form, but as a mono-alkali metal salt. Moreover, based on the result of single crystal X-ray diffraction, reported is the structure of PQQ in the free form (Nature, vol. 280, 30 August, 1979, P844), which, in fact, includes PQQ-acetone adduct(s).

Methods such as use of ion exchange resins and extraction with organic solvent following acidification are common for converting an alkali metal salt of a compound into the free compound. In the case of using ion exchange resins, however, poor solubility of PQQ in the free form requires a large amount of extraction liquid, which in turn also requires a subsequent concentration step, thereby rendering the process inefficient. Moreover, in the extraction with an organic solvent, the extraction device is made applicable to organic solvents and thus expensive. Furthermore, it is preferred to minimize the amount of the organic solvent because the larger the amount, the higher the risk for fire and explosion.

### SUMMARY OF THE INVENTION

The present inventors have found that pyrroloquinoline quinone in the free form is precipitated by adjusting the pH of a solution containing a dissolved alkali metal salt of pyrroloquinoline quinone to 1.5 or less (Examples 1 to 3). The present invention is based on this finding.

An object of the present invention is to provide a method for conveniently producing pyrroloquinoline quinone in the free form without any organic solvent or ion-exchange resin and highly-pure crystals thereof.

According to the present invention, there is provided a production method of the compound represented by formula (1): 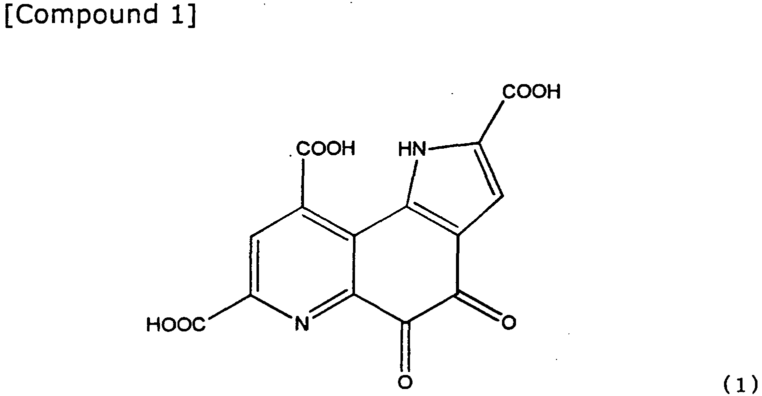 (pyrroloquinoline quinone in the free form), wherein the method (hereinafter sometimes referred to as "the production method according to the present invention") comprises preparing a solution having a pH of 1.5 or less by dissolving an alkali metal salt of the compound to obtain a precipitate.

According to the present invention, there is also provided the compound represented by formula (1): 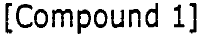 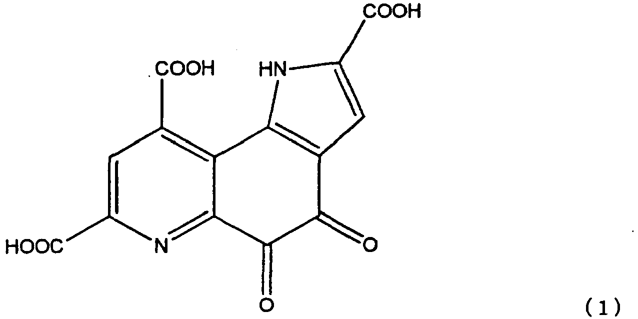 (hereinafter sometimes referred to as "the compound according to the present invention") which is produced by the production method according to the present invention.

According to the present invention, there are further provided crystals of the compound represented by formula (1): 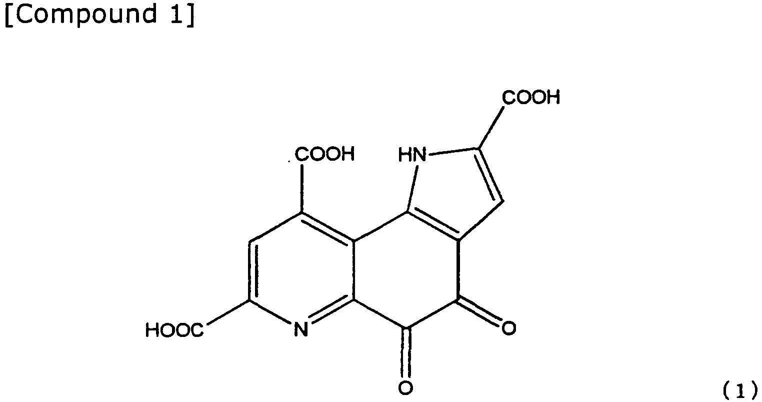 (hereinafter sometimes referred to as "the crystals according to the present invention").

According to the present invention, pyrroloquinoline quinone in the free form having a high purity because of a low alkali metal content and good crystallinity can be conveniently produced advantageously without any organic solvent or ion-exchange resin.

The pyrroloquinoline quinone in the free form obtained according to the present invention may be an active ingredient in medicines or functional foods and advantageously provided in forms such as topical dermatological agents, injections, oral agents and suppositories, or in forms such as daily foods and drinks, nutrition-enriched diets and various hospital diets.

### BRIEF DESCRIPTION OF THE DRAWINGS

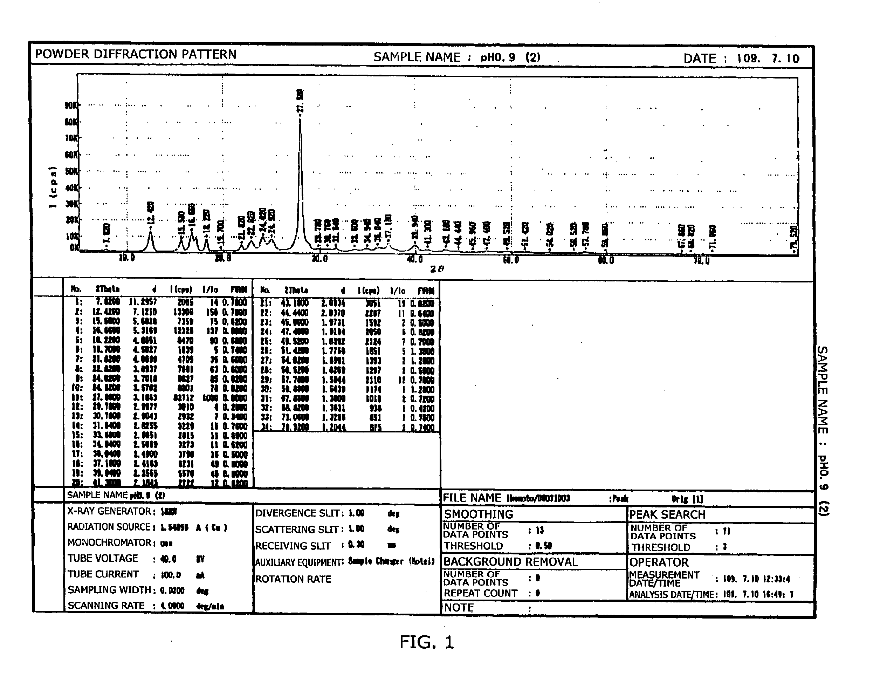

[Figure 1] Figure 1 shows the analysis result of X-ray powder diffraction pattern of PQQ in the free form obtained in Example 1.

### DETAILED DESCRIPTION OF THE INVENTION

According to the present invention, pyrroloquinoline quinone in the free form can be produced by preparing a solution having a pH of 1.5 or less by dissolving an alkali metal salt of pyrroloquinoline quinone to obtain a precipitate.

The terms "pyrroloquinoline quinone" and "pyrroloquinoline quinone in the free form" as used herein refer to the compound represented by formula (1) and both terms are used interchangeably to refer to the compound.

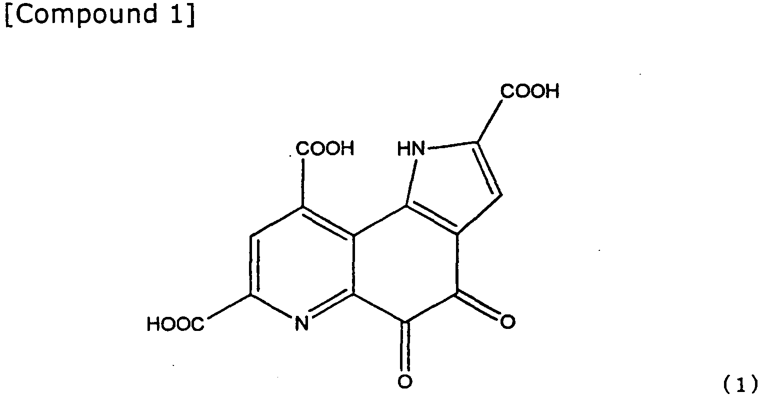

The term "alkali metal salt of pyrroloquinoline quinone" as used herein refers to an alkali metal salt of the compound represented by formula (1).

The term "crystals of pyrroloquinoline quinone in the free form" as used herein refers to crystals of the compound represented by formula (1), which are in the state where pyrroloquinoline quinone molecules are regularly arranged in the solid state and generally are highly pure and highly stable. Whether or not the compound of formula (1) (pyrroloquinoline quinone in the free form) is in a crystalline form can be determined by X-ray powder diffraction through peak detection.

The production method according to the present invention can be achieved by carrying out a step of preparing a solution having a pH of 1.5 or less by dissolving an alkali metal salt of pyrroloquinoline quinone to obtain a precipitate.

In the present invention, the alkali metal salt of pyrroloquinoline quinone used as a raw material for producing pyrroloquinoline quinone in the free form includes salts of sodium, potassium, lithium, cesium, rubidium, and the like. These salts each may be used singly or in combination. Preferred are single sodium and potassium salts, and sodium salt is more preferred because it is easily purchased. Pyrroloquinoline quinone may be substituted with one to three alkali metals to form an alkali metal salt thereof, which may be any of a monoalkali metal salt, a dialkali metal salt and a trialkali metal salt, preferably a dialkali metal salt. The alkali metal salt of pyrroloquinoline quinone is especially preferably a disodium salt.

PQQ in the free form can be produced by using such an alkali metal salt of PQQ and allowing precipitation to occur in an aqueous solution having a pH of 1.5 or less. In a similar manner, it is also possible to produce the PQQ in the free form likewise from an alkaline-earth metal salt, an ammonium salt, an organic ammonium salt, and a transition metal salt.

The alkali metal salt of pyrroloquinoline quinone used as a raw material in the present invention may be commercially available.

The alkali metal salt of pyrroloquinoline quinone used as a raw material in the present invention can be produced by known methods such as organic chemical syntheses and fermentation processes.

The alkali metal salt of pyrroloquinoline quinone used as a raw material in the present invention may be crystalline or amorphous. Moreover, it may have some impurities.

When used in solution, the alkali metal salt of pyrroloquinoline quinone can be dissolved in a solvent such as water and an alcohol to form a solution. Preferably, the alkali metal salt of pyrroloquinoline quinone is used as an aqueous solution.

The solution of the alkali metal salt of pyrroloquinoline quinone can be prepared in a concentration of, for example, 0.001 to 1% w/w, preferably 0.005 to 0.5% w/w, more preferably 0.01 to 0.25% w/w.

When an alkali metal salt of pyrroloquinoline quinone is used in solution, the pH of the solution may be adjusted to 3 or more and 13 or less, preferably 5 to 12, more preferably 7 to 10. To adjust the pH of the solution, an alkaline substance (for example, sodium hydroxide) can be added to the solution. The then temperature of the solution may be 0 to 140°C. Preferably, the solution at a temperature of 20 to 90°C is easily used in no need of special equipment. Since the solubility tends to increase with temperature, the solution used is preferably at a temperature of 50°C or more from the viewpoint of productivity.

In the production method according to the present invention, a solution having a pH of 1.5 or less obtained by dissolving an alkali metal salt of pyrroloquinoline quinone is prepared to allow the target pyrroloquinoline quinone in the free form to precipitate.

Preparation of the solution having a pH of 1.5 or less may be performed by using an acid.

The pH of the solution may be 1.5 or less, preferably 1 or less. The alkali metal may remain in the precipitates from a solution having a pH of 2 or more; that is, a mono- or higher sodium salt may be formed. It is preferable to use a solution having a pH of 1.5 or less to prevent formation of such salts, and more preferable to allow precipitation to occur in a solution having a pH of 1 or less. If the pH of the solution is not sufficiently low during precipitation, the precipitation crystals form a solid having the peaks attributable to the structure of a mono-alkali metal salt. Therefore, the pH of the solution is desirably 1 or less for improving crystallinity.

In the production method according to the present invention, the pH of the solution may be 0.5 to 1.5, preferably 0.5 to 1, more preferably 0.6 to 0.9.

In the production method according to the present invention, the step of preparing a solution having a pH of 1.5 or less by dissolving an alkali metal salt of pyrroloquinoline quinone can be carried out by adding an acid to a solution of an alkali metal salt of pyrroloquinoline quinone to lower the pH of the solution, or adding a solution of an alkali metal salt of pyrroloquinoline quinone to a solution of an acid. Moreover, an operation such as recrystallization may also be carried out by mixing an alkali metal salt of pyrroloquinoline quinone in the solid form with an acid. This uses only a small amount of water and is convenient, although the alkali metal tends to remain because a mixed state does not occur homogenously when the acid is added rapidly.

Specifically, a solution having a pH of 1.5 or less containing an alkali metal salt of pyrroloquinoline quinone can be prepared by carrying out the following steps (hereinafter sometimes referred to as "preparation steps") of:
(i) adding an acid or an acidic solution to a solution obtained by dissolving an alkali metal salt of pyrroloquinoline quinone to adjust the pH of the solution to 1.5 or less;
(ii) adding an alkali metal salt of pyrroloquinoline quinone or a solution obtained by dissolving an alkali metal salt of pyrroloquinoline quinone to an acidic solution to adjust the pH of the solution to 1.5 or less; or
(iii) adding a solution obtained by dissolving an alkali metal salt of pyrroloquinoline quinone to an acid to adjust the pH of the solution to 1.5 or less.
Preferably, the solution having a pH of 1.5 or less containing an alkali metal salt of pyrroloquinoline quinone can be prepared by carrying out the step of adding an acid or an acidic solution to a solution obtained by dissolving an alkali metal salt of pyrroloquinoline quinone to adjust the pH of the solution to 1.5 or less.

The term "addition" as used herein means an additive may be added at once or gradually to an object to which the additive is to be added.

Any kind of acid that can lower the pH is used in the preparation steps. Either inorganic acids or organic acids can be used. Specifically, inorganic acids includes hydrochloric acid, hydrogen bromide, hydrogen iodide, perchloric acid, sulfuric acid, phosphoric acid nitric acid and the like, with hydrochloric acid, hydrogen bromide, hydrogen iodide, perchloric acid, phosphoric acid and nitric acid being preferred and hydrochloric acid being more preferred. Organic acids includes acetic acid, formic acid, oxalic acid, lactic acid, citric acid and the like, with acetic acid, formic acid, oxalic acid, lactic acid and citric acid being preferred. The risk exists that the acid may remain in pyrroloquinoline quinone because the pH used is low. Therefore, a nontoxic monovalent acid having a certain vapor pressure, for example, hydrochloric acid is most preferred. Hydrochloric acid is also excellent in crystallinity in that it provides a solid as defined in the scope of the present invention. A polyvalent acid forms a salt with an alkali metal ion, which often has a low solubility, and then tends to remain in the resultant solid. Sulfuric acid, which is easily used with no fuming, tends to remain in the resultant solid, and the risk exists that discoloration may be caused by the acid's dehydrating property. Therefore, it is preferred to use sulfuric acid in combination with one or more other acids.

The acid can be used singly or in combination with one or more other acids. It is preferred, however, to use at least hydrochloric acid, that is, to use hydrochloric acid singly or in combination with one or more other acids (for example, hydrogen bromide, hydrogen iodide, perchloric acid, sulfuric acid, phosphoric acid, nitric acid and the like).

The acid can be used in diluted form (acidic solution). The acid can be dissolved in a solvent such as water and used as an acidic solution.

The acidic solution can be prepared in a concentration of, for example, 0.1 to 20 g/L, preferably 0.5 to 10 g/L.

The molar ratio of an alkali metal salt of pyrroloquinoline quinone to an acid may be 1: 1 to 1: 1000, preferably 1: 2 to 1: 100.

The preparation step may be carried out at any temperature. It may, however, be carried out at a temperature of between -20°C and 140°C from the viewpoint of ease of general handling. When attempting to increase the solubility of the alkali metal salt of pyrroloquinoline quinone, warming may be conducted.

The preparation step may be carried out for any period of time. It may, however, be carried out for between 5 minutes and about one week. Only a short time is required on a small scale, while a longer time on a large scale.

From the solution having a pH of 1.5 or less obtained by dissolving an alkali metal salt of pyrroloquinoline quinone, prepared in the preparation step mentioned above, a precipitate can be obtained.

The term "precipitate" as used herein refers to a solid phase (solid) formed from a liquid phase (solution).

In the production method according to the present invention, a step of allowing to stand the solution having a pH of 1.5 or less obtained by dissolving an alkali metal salt of pyrroloquinoline quinone, prepared in the preparation step mentioned above, so as to obtain a precipitate (hereinafter sometimes referred to as "precipitation step") can be carried out. In the step, the solution may be stirred.

The stirring may be carried out by subjecting the solution to magnetic stirring, mechanical stirring, manual stirring and shake stirring, preferably magnetic stirring or mechanical stirring.

The precipitation step may be carried out at any temperature. It may, however, be carried out at -20°C to 100°C from the viewpoint of ease of general handling, and preferably at a temperature between -10 and 70°C, more preferably between 0 and 50°C because of ease of precipitating at a lower temperature in precipitation.

The precipitation step may be carried out for any period of time. It is, however, carried out for between one minute and one week, preferably between 30 minutes and one hour.

The precipitated solid (precipitate) may be separated from the liquid by known methods such as filtration and centrifugation. This solid may be washed with water, hydrochloric acid solution and, if necessary, an organic solvent such as isopropanol. The resultant solid may be subjected to air drying and drying under reduced pressure to remove the moisture.

More specifically, the production method according to the present invention may be carried out as follows:
An alkali metal salt of pyrroloquinoline quinone is dissolved in water. This solution has desirably a pH of 3 or more and 13 or less, more preferably between 5 and 12. In this case, the pH of the solution may be adjusted by adding an alkaline solution. The then temperature of the solution may be between 0 and 140°C. Preferably, the solution at a temperature of between 20 and 90°C is easily used in no need of special equipment. Since the solubility tends to increase temperature, the solution at a temperature of 50°C or more improves productivity. To this solution, an acid is added to lower the pH of the solution to the range of the present invention, allowing a solid to precipitate. Alternatively, the alkali metal salt solution may be added to an acidic solution, allowing precipitation to occur. The precipitate may also be produced by adding a solid alkali metal salt in powder form to an acidic solution. If the admixing to reduce the pH is performed while stirring, it is possible to prevent the alkali metal from remaining in the precipitate.

The solid that has precipitated after the preparation under such conditions is pyrroloquinoline quinone in the free form, and is in the state of pyrroloquinoline quinone crystals which exhibit peaks at 2θ of 12.4°, 15.5°, 16.6°, 18.2°, 24.0°, 24.9° and 28.0° (any are within ±0.2°) in the powder X-ray diffraction using Cu Kα radiation.

### <Conditions for measuring the diffraction angle 2θ by powder X-ray diffraction>

Instrument: M 18XCE from MAC Science Corporation
X-ray: Cu/Tube voltage 40 kV/Tube current 100 mA
Divergence Slit: 1°
Scattering Slit: 1°
Receiving Slit: 0.3 mm
Scanning Rate: 4.000°/min
Sampling Width: 0.020°

In addition, these peaks may also be observed on any other common powder X-ray diffraction instrument equipped with a monochromator. The crystalline forms defined in the present invention have only to be reasonably identical in the peak angle since a measurement error is included.

According to a preferred embodiment of the present invention, there is provided a production method of the compound represented by formula (1) (pyrroloquinoline quinone in the free form), comprising adding an acid or an acidic solution to an aqueous solution obtained by dissolving an alkali metal salt of the compound represented by formula (1) to prepare a solution having a pH of 1.5 or less and obtaining a precipitate, wherein the alkali metal salt is preferably selected from the group consisting of sodium salt, potassium salt, lithium salt, cesium salt and rubidium salt, the acid is preferably one or a combination of two or more selected from the group consisting of hydrochloric acid, hydrogen bromide, hydrogen iodide, perchloric acid phosphoric acid, nitric acid, acetic acid, formic acid, oxalic acid, lactic acid and citric acid, and the pH is preferably one or less.

According to a preferred embodiment of the present invention, there is also provided a production method of the compound represented by formula (1) (pyrroloquinoline quinone in the free form), comprising adding to an acidic solution an alkali metal salt of the compound represented by formula (1) or an aqueous solution obtained by dissolving the alkali metal salt to prepare a solution having a pH of 1.5 or less and obtaining a precipitate, wherein the alkali metal salt is preferably selected from the group consisting of sodium salt, potassium salt, lithium salt, cesium salt and rubidium salt, the acid is preferably one or a combination of two or more selected from the group consisting of hydrochloric acid, hydrogen bromide, hydrogen iodide, perchloric acid, phosphoric acid, nitric acid, acetic acid, formic acid, oxalic acid, lactic acid and citric acid, and the pH is preferably one or less.

According to a preferred embodiment of the present invention, there is provided a production method of the compound represented by formula (1) (pyrroloquinoline quinone in the free form), comprising adding an acid or an acidic solution to an aqueous solution obtained by dissolving an alkali metal salt of the compound represented by formula (1) to prepare a solution having a pH of 1.5 or less and obtaining a precipitate, wherein the alkali metal salt is preferably selected from the group consisting of sodium salt, potassium salt, lithium salt, cesium salt and rubidium salt, the acid is preferably one acid or a combination of two or more acids including at least hydrochloric acid, and the pH is preferably one or less.

The pyrroloquinoline quinone in the free form obtained in the present invention has a high purity because of a low alkali metal content and good crystallinity. The diffraction peaks from this free form can be distinguished from those from disodium salt and monosodium salt, enabling effective quality control.

The pyrroloquinoline quinone in the free form obtained by the production method according to the present invention may be an active ingredient in medicines or functional foods and provided in forms such as topical dermatological agents, injections, oral agents and suppositories, or forms such as daily foods and drinks, nutrition-enriched diets and various hospital diets. The additive used in the preparation may include water, sugars such as fructose and glucose, oils such as peanut oil, soybean oil and olive oil, and glycols such as polyethylene glycol and polypropylene glycol.

As for solid preparations such as tablets, capsules and granules, examples of excipients include sugars such as lactose, sucrose and mannitol, lubricants include kaolin, talc and magnesium stearate, disintegrants include starch and sodium alginate, binders include polyvinyl alcohol, cellulose and gelatin, surfactants include fatty acid ester, and plasticizers include glycerin. The examples are not limited to those cited above. Solubility enhancing agents and fillers may be added if necessary.

Moreover, pyrroloquinoline quinone in the free form may be used alone or in combination with other materials. Examples of the material that may be used in combination include, but not limited to vitamins such as vitamin B complex, vitamin C and vitamin E, amino acids, astaxanthin, carotenoids such as α-carotene and β-carotene, ω-3 fatty acids such as docosahexaenoic acid and eicosapentaenoic acid, and ω-6 fatty acids such as arachidonic acid.

According to the present invention, the following inventions are further provided.
(1) A production method of pyrroloquinoline quinone in the free form, characterized in that an alkali metal salt of pyrroloquinoline quinone is allowed to precipitate in an aqueous solution having a pH of 1.5 or less.
(2) The production method of (1), characterized in that the alkali metal salt of pyrroloquinoline quinone is selected from sodium salt, potassium salt, lithium salt, cesium salt and rubidium salt.
(3) The production method of (1), characterized in that the alkali metal salt of pyrroloquinoline quinone is a disodium salt.
(4) The production method of (1) to (3), characterized in that an acid used to precipitate pyrroloquinoline quinone is selected from hydrochloric acid, hydrogen bromide, hydrogen iodide, perchloric acid, sulfuric acid, phosphoric acid, nitric acid, acetic acid, formic acid, oxalic acid, lactic acid and citric acid.
(5) Pyrroloquinoline quinone crystals exhibiting peaks at 2θ of 12.4°, 15.5°, 16.6°, 18.2°, 24.0°, 24.9° and 28.0° in the powder X-ray diffraction using Cu Kα radiation.

### EXAMPLES

The present invention will now be described more specifically with reference to the following examples and comparative examples, but is not intended to be limited thereto. In addition, all percentages in the context of the present invention are by weight, unless otherwise stated.

Analyses for the present invention were performed as follows:

### [PQQ Analysis]

Instrument: SHIMADZU CORPORATION, High Performance Liquid Chromatography, LC-20A
Column: YMC-Pack ODS-TMS (5 µm), 150x4.6 mm I.D.
Measurement Temperature: 40°C
Detection: Absorbance at 260 nm
Eluent: 100 mM CH₃COOH/100 mM CH₃COONH₄ (30/70, pH 5.1)
Elution Rate: 1.5 mL/min

### [Na Analysis]

Pump: SHIMADZU CORPORATION, LC6A
Column Oven: SHIMADZU CORPORATION, HIC-6A
Measurement Temperature: 40°C
Detector: Tosoh CORPORATION, Electroconductivity
Detector CM-8000
Column: Showa Denko K.K., Shodex IC Y-521
Eluent: 4 mM HNO₃
Elution Rate: 1.0 mL/min

### [Powder X-ray Diffraction]

Instrument: M18XCE from MAC Science Corporation
X-ray: Cu/Tube voltage 40 kV/Tube current 100 mA
Divergence Slit: 1°
Scattering Slit: 1°
Receiving Slit: 0.3 mm
Scanning rate: 4.000°/min
Sampling Width: 0.02

### Example 1

A reagent (trade name: BioPQQ) from MITSUBISHI GAS CHEMICAL COMPANY, INC. was used as the raw material PQQ disodium salt. The PQQ disodium salt had a purity of 99.0% as determined by UV absorption on high performance liquid chromatography.

To 198 g of water, 2 g of the disodium salt mentioned above were added to obtain an aqueous solution of disodium salt. To the resultant solution, NaOH was added to adjust the pH to 9. To this solution, 7.7 g of a solution obtained by subjecting concentrated hydrochloric acid (from Wako Pure Chemical Industries, Ltd.) to dilution by 50% with water were added to adjust the pH to 0.9. After a 30-minute stirring, the precipitated solid was filtered and washed with water and isopropanol. This substance was dried at 50°C and a reduced pressure overnight. The red crystals collected weighed 1.6 g. The Na analysis shows that PQQ in the free form having a Na content of 0 and thus containing no sodium was obtained by the simple method. The result of powder X-ray diffraction for the resultant PQQ in the free form is shown in Fig. 1. The PQQ in the free form exhibited peaks at 2θ of 12.4°, 15.5°, 16.6°, 18.2°, 24.0°, 24.9° and 28.0° in the powder X-ray diffraction using Cu Kα radiation.

### Example 2

The raw material in Example 1 (PQQ disodium salt) was dissolved in water. To the solution, sodium hydroxide was added to adjust the pH to 8, followed by addition of sodium chloride for precipitation of PQQ trisodium salt. The precipitated PQQ trisodium salt was then washed with ethanol and dried. This salt was used in the subsequent experiment.

In water (60 g), 0.9 g of the PQQ trisodium salt was dissolved. To this were added about 2 g of concentrated hydrochloric acid while stirring. The resultant solution had a pH of 0.6. After overnight stirring, the solution was filtered, and the residue was washed with isopropanol and dried under reduced pressure to obtain 0.35 g of red solid. The results of powder X-ray diffraction and Na analysis for the red solid obtained were similar to those in Example 1, and indicated no residual sodium in the red solid. It was shown to be pyrroloquinoline quinone crystals in the free form.

### Example 3

To a mixed solution of 3.5 g of concentrated hydrochloric acid and 3.5 g of water, 1 g of the same PQQ disodium salt solid as one in Example 1 was added to adjust the pH of the solution to 1. After stirring at room temperature for one hour, the solution was filtered. The residue was washed with water and dried under reduced pressure to obtain 0.79 g of red solid. The molar ratio of sodium to PQQ in the resultant red solid was 0.06, indicating that a small amount of sodium remained in the solid.

### Comparative Example 1

The same raw material (PQQ disodium salt) as one in Example 1 was used, and the operations were carried out as in Example 1, except that hydrochloric acid is added to the solution to adjust the pH of the solution to 2.3, allowing 1.75 g of red solid to precipitate.

The results of the analyses for this substance showed that the molar ratio of sodium to PQQ in the substance was 0.96, indicating that the substance was PQQ monosodium salt. The powder X-ray diffraction data for this monosodium salt showed peaks at 2θ of 8.5°, 11.9°, 15.7°, 16.9°, 24.4° and 27.3° in the powder X-ray diffraction using Cu Kα radiation. These peaks were different from those from the PQQ in the free form.

### Comparative Example 2

The operations similar to those in Comparative Example 1 were carried out, except that 4N sulfuric acid was used to adjust the pH of the solution to 2.5, affording 1.71 g of red solid.

The results of the analyses for this substance showed that the molar ratio of sodium to PQQ in the substance was 0.94 and that the substance was PQQ monosodium salt. The powder X-ray diffraction data for this monosodium salt showed peaks at the same positions as those in Comparative Example 1.

### Comparative Example 3

The same raw material (PQQ disodium salt) as one in Example 1 was used as in the solid form to perform this experiment. To 200 g of 4N sulfuric acid, 2 g of PQQ disodium salt were added to adjust the pH of the solution to 0.6, affording 1.66 g of brown solid.

The results of the analyses for this substance showed that the molar ratio of sodium to PQQ in the substance was 0.96, in the range of which the substance should be PQQ monosodium salt. The powder X-ray diffraction data revealed a spectrum having no well-defined peaks, suggesting the substance's low crystallinity. The spectrum showed a broad peak centering about 26.6° as a peak at 2θ in the powder X-ray diffraction using Cu Kα radiation. In this case, the substance had residual sodium, underwent discoloration, and had low crystallinity.

## Claims

1. A production method of the compound represented by formula (1): 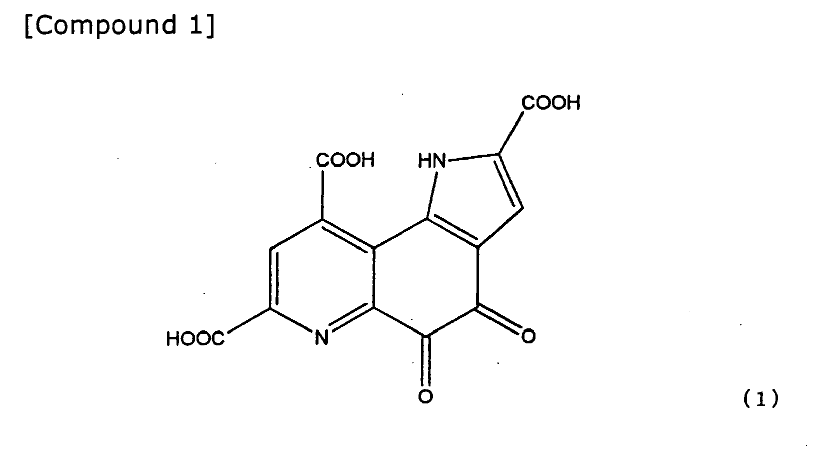 comprising preparing a solution having a pH of 1.5 or less by dissolving an alkali metal salt of the compound to obtain a precipitate.

2. The production method of claim 1, wherein the alkali metal salt is selected from sodium salt, potassium salt, lithium salt, cesium salt and rubidium salt.

3. The production method of claim 1, wherein the alkali metal salt is a dialkali metal salt.

4. The production method of claim 1, wherein the alkali metal salt is a disodium salt.

5. The production method of claim 1, wherein the solution having a pH of 1.5 or less is prepared by using an acid.

6. The production method of claim 5, wherein the acid is one or a combination of two or more selected from hydrochloric acid, hydrogen bromide, hydrogen iodide, perchloric acid, sulfuric acid, phosphoric acid, nitric acid, acetic acid, formic acid, oxalic acid, lactic acid and citric acid.

7. The production method of any one of claims 1 to 6, wherein the compound represented by formula (1) is in a crystalline form.

8. A compound represented by formula (1): 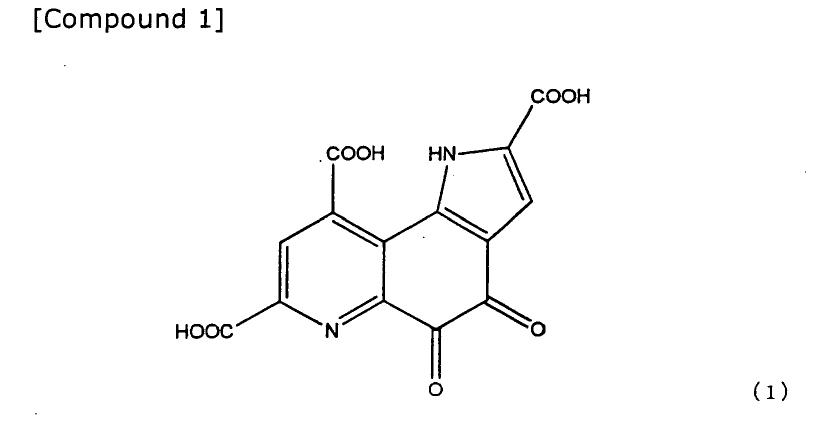 produced by the production method of any one of claims 1 to 7.

9. A crystal of the compound represented by formula (1):

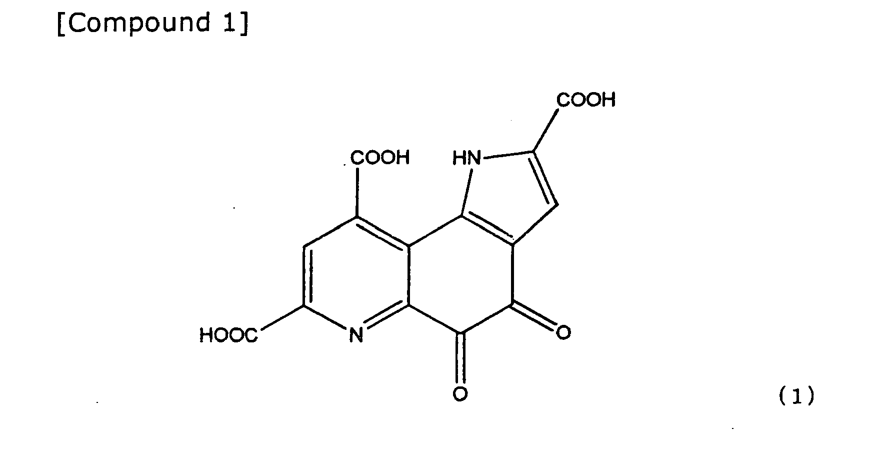

10. The crystal of claim 9, exhibiting peaks at 2θ of 12.4°, 15.5°, 16.6°, 18.2°, 24.0°, 24.9° and 28.0° (any are within ±0.2°) in the powder X-ray diffraction using Cu Kα radiation.
